# EUROPEAN PATENT APPLICATION

(11) **EP 1 338 256 A1**
(43) Date of publication of application: **27.08.2003**
(21) Application number: 03251139.6
(22) Date of filing: 26.02.2003
(51) Int. Cl.: A61F 2/34, B26F 1/36, B25B 7/00, B26F 1/40, A61F 2/46

(54) **Acetabular component with removable screw hole plugs**

(30) Priority: 26.02.2002 US 82957
(71) Applicant: DePuy Products, Inc., Warsaw, IN 46581 (US)
(72) Inventor: Nycz, Jeffrey H., Warsaw, Indiana (US); Jones, Michael C., North Webster, Indiana (US); Brown, Scott C., Warsaw, Indiana (US); Rhodes, Joel, Pierceton, Indiana (US)
(74) Representative: Belcher, Simon James

(57) **Abstract**

An articulating hemiarthroplasty prosthesis (10) for implantation into the human anatomy (14) is provided. The prosthesis includes a cup (12) for engagement with the human anatomy (14). The cup (12) has a portion (64) having a reduced thickness (T₂) to permit at least a portion of the cup (12) to be mechanically separated from the cup (12) to form an opening (66) through the cup (12)

## Description

The present invention relates generally to the field of orthopaedics, and more particularly, to an implant for use in arthroplasty.

The invention relates to implantable articles and methods for manufacturing such articles. More particularly the invention relates to bone prostheses.

There are known to exist many designs for and methods for implanting articles such as bone prosthesis. Such bone prosthesis include components of artificial joints such as elbows, hips, knees, and shoulders. An important consideration in the design and implantation of virtually any implantable bone prosthesis is that the prosthesis has adequate fixation when implanted within the body.

Early designs of implantable articles relied upon the use of cements such as polymethylmethacrylate to anchor the implant. The use of such cements can have some advantages, such as providing a fixation that does not develop free-play or does not lead to erosion of the joining bone faces postoperatively. However, the current trend is to use these cements to a lesser extent because of their tendency to lose adhesive properties over time and the possibility that cement contributes to wear debris within a joint.

Recently, implantable bone prosthesis had been designed such that they encourage the growth of hard tissue (i.e., bone) around the implant. The bone attachment usually occurs and growth is promoted when the surface of the implantable bone prosthesis is irregular or textured. The interaction of newly formed hard tissue in and around the textured surface of the implantable bone prosthesis has been found to provide a good fixation of the prosthesis within the body. A greater degree of bone fixation can usually be achieved when the bone engaging surface of an implantable bone prosthesis are more porous or irregular.

A porous or irregular surface can be provided in implantable articles by a variety of techniques. In some instance, an irregular surface pattern or surface porosity is formed in an implantable bone prosthesis by embossing, chemical etching, milling or machining.

One very important type of bone prosthesis is an articulating hemiarthroplasty prosthesis. Such prosthesis is used extensively for hips and shoulders. By far the most common of these articulating hemiarthoplasty prostheses are hip prostheses.

Hip prostheses typically include a first component in the form of a stem which is fitted within the medullary canal of the femur and a cup component that is secured to the acetabulum. The stem component typically has a spherical or bowl-shaped component on the proximal end and the cup includes an internal surface which is generally spherical. The cup and the ball may directly mate or more commonly include a liner or plastic insert which is positioned between the cup and the ball.

The hip cup may be secured to the acetabulum in one of several various ways. For example, the hip cup might be an interference fit in a reamed opening of the acetabulum. Alternatively, the hip cup may be cemented to the acetabulum by use of cement such as polymethylmethacrylate (PMMU). Alternatively, the hip cup may be secured to the acetabulum by use of porous or irregular surface that promote and encourage the growth of hard tissue in the form of bone around the implant. Alternatively, the hip cup may include threads on the external periphery thereof which are self-tapping and may be used to threadably engage the acetabulum.

Another very common method of securing a hip cup to the acetabulum is by the use of a bone screw which is fitted through an opening in the hip cup. Depending on the anatomy of the particular patient, including the shape of the patient's acetabulum as well as the location of healthy bone tissue, the optimum position of the opening or openings in the hip cup may vary widely from patient to patient.

Two solutions have been developed for providing prosthetic hip cups for use with self-tapping screws. One type of hip cup is a special or individual cup which is designed for a particular patient. The proper position for the holes to accommodate a hip screw or screws is determined by x-ray or other techniques upon the patient and a suitable hip cup is individually designed and manufactured for the patient. This method is very expensive.

An alternate solution to providing for hip cups for use with self-tapping hip screws is the use of a hip cup including a multitude of holes or openings. Only those holes or openings which are in alignment with a suitable portion of the acetabulum of the patient are utilized. The other holes are not used and are not thus plugged by a screw.

Attempts have been made to plug the unused holes of a prosthetic hip cup. For example, the unused hip cup holes may be plugged by a plastic insert. Such an insert is snap-fitted into the opening. Another solution to plugging the hip screw holes is to provide for threads on the periphery of the hip screw holes and to provide for threaded plugs , typically metal plugs, to fill the holes.

The use of plastic plugs and threaded metal plugs has a plaguing problem. Neither the plastic plugs nor the threaded metal plugs provides for adequate sealing of synodical fluid.

Neither the polyethylene plugs nor the machined titanium inserts are adequate in sealing polyethylene wear particles from migrating to the acetabulum. Synodical fluid may thus be contaminated by polyethylene wear particles. The contamination of polyethylene wear particles at the location of bone, particularly the acetabulum, may lead to osteolysis. Osteolysis is the loss of bone due to contamination. Such loss of bone may lead to joint loosening and may require a revision or replacement of the implant.

The present invention is directed to alleviate at least some of the aforementioned problems.

Accordingly, a need has arisen for a prosthesis which provides occlusion of the screw holes in acetabular components while addressing the issue of sealing polyethylene wear particles from migrating to the acetabulum.

The present invention provides for a true seal of screw holes by a special machining and tooling process. This seal remains an integral part of the component until removed through special instrumentation.

A technical advantage of the present invention includes the complete sealing of the screw hole plugs and of the acetabulum from synodical fluid and polyethylene wear debris, while providing a method for removal of additional openings within the hip cup for removal in cases where additional fixation is required.

Another technical advantage of the present invention is the availability of a multitude of unique hip cups available from an identical hip cup product code. The various hip cups would be made or provided by machining additional openings into the common hip cup. Such an ability to utilize a common hip cup provides for larger production lots, reduced per unit manufacturing cost and lower inventory as well as a fewer number of product codes.

According to one embodiment of the present invention, there is provided an articulating hemiarthroplasty prosthesis for implantation into the human anatomy. The prosthesis includes a cup for engagement with the human anatomy. The cup has a portion having a reduced thickness to permit at least a portion of the cup to be mechanically separated from the cup to form an opening through the cup.

According to another embodiment of the present invention there is provided a tool for use with an articulating hemiarthroplasty cup prosthesis to remove a portion of the cup. The portion has a first cup surface and a second opposed cup surface. The tool includes a first component having a first tool surface adapted to conform with the first cup surface and a second component. The second component has a second tool surface adapted to conform with the second cup surface. The first component and the second component cooperate with each other and are adapted to remove the portion of the cup by placing the portion of the cup between the first component and the second component and by advancing the first component toward the second component.

According to yet another embodiment of the present invention there is provided a cup for engagement with the human anatomy for use in an articulating hemiarthroplasty prosthesis for implantation into the human anatomy. The cup includes a portion of the cup which has a reduced thickness to permit at least a portion of the cup to be mechanically separated from the cup to form an opening through the cup

According to another embodiment of the present invention there is provided a hip prosthesis for implantation into the human anatomy. The prosthesis includes a cup for engagement with the acetabulum. The cup includes a portion of the cup which has a reduced thickness to permit at least a portion of the cup to be mechanically separated from the cup to form an opening through the cup. The portion of the cup which has the reduced thickness is adapted to block the flow of synodical fluid through the cup.

According to a further embodiment of the present invention, there is provided a method for providing total hip arthroplasty including the steps of providing an acetabulum hip screw, providing a cup with a plurality of potential mounting portions thereof each portion having a reduced cross section, determining a mounting location on the acetabulum which will accommodate an acetabulum hip screw, aligning one of the mounting portions with the mounting location, removing at least a portion of one of the mounting portions to form an opening through the cup, placing the acetabulum hip screw into the opening, and securing the cup to the acetabulum by screwing the hip screw into the acetabulum.

Embodiments of the invention will now be described by way of example with reference to the accompanying drawings, in which:
FIGURE 1 is a perspective view of a hip prosthesis cup for implanting into an acetabulum in accordance with an embodiment of the present invention;
FIGURE 2 is a cross-sectional view of the hip cup along the line 2-2 of Figure 1 in the direction of the arrows in accordance with the embodiment of the present invention of Figure 1;
FIGURE 3 is a plan view of a punch pliers for use with hip cup of Figure 1 with the punch pliers in engagement with the hip cup of Figure 1;
FIGURE 4 is an enlarged plan view of the punch pliers of Figure 3 in engagement with the hip cup of Figure 1;
FIGURE 5 is an enlarged plan view of the punch pliers of Figure 3 in engagement with a portion of the hip cup of Figure 1;
FIGURE 6 is a partial cross-sectional plan view of the hip cup of Figure 2 in cooperation with the punch pliers of Figure 3 in accordance with an embodiment of the present invention showing the shearing punch die spaced from the hip cup;
FIGURE 7 is a partial cross-sectional plan view of the hip cup of Figure 2 and the punch pliers of Figure 3 in accordance with an embodiment of the present invention showing the shearing punch die in contact with the hip cup;
FIGURE 8 is a partial plan view of the hip cup of Figure 2 and the punch piers of Figure 3 in accordance with an embodiment of the present invention showing the shearing punch die having sheared a portion of the hip cup from the hip cup resulting in a screw hole according to the present invention;
FIGURE 9 is a cross-sectional view of the hip cup along the line 9-9 of Figure 1 in the direction of the arrows in accordance with the embodiment of the present invention of Figure 1 showing a hip screw placed in the screw hole;
FIGURE 10 is a partial perspective view of the punch pliers of Figure 3 for use with hip cup of Figure 1;
FIGURE 11 is a partial cross-sectional view of an alternate embodiment of the hip prosthesis cup of the present invention for implanting into an acetabulum having an undercut for ease of removal of an opening;
FIGURE 12 is a plan view of a hip prosthesis including the hip cup of Figure 1;
FIGURE 13 is a process flow chart for a method of providing total hip arthroplasty according to the present invention;
FIGURE 14 is a plan view of an alternate punching tool for use with the hip cup of Figure 1;
FIGURE 14A is a plan view of the alternate punching tool for use with the hip cup of Figure 1; and
FIGURE 15 is a partial cross-sectional view of another alternate punching tool for use with the hip cup of Figure 1.

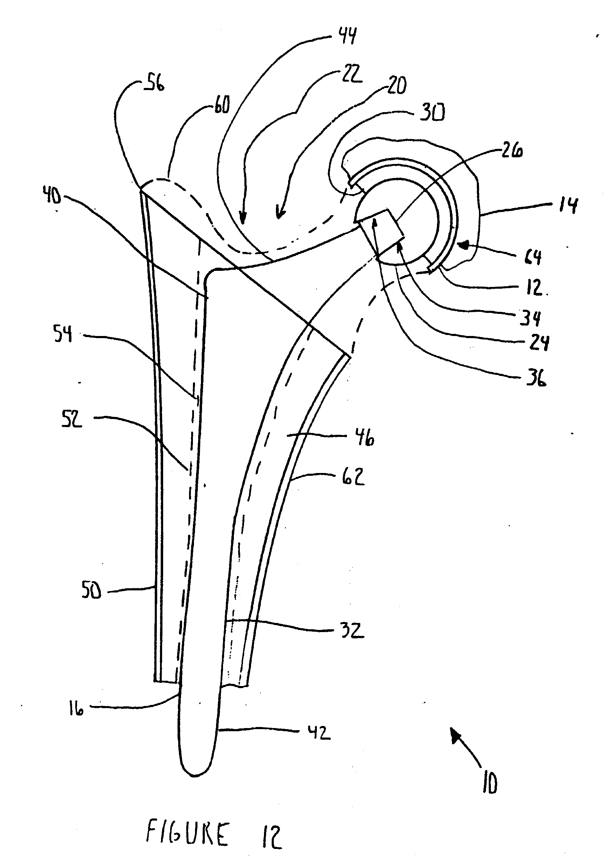

Referring to the drawings, Figure 12 shows an articulating hemiarthroplasty prosthesis 10 for implantation into the human anatomy. The prosthesis 10 includes a cup 12 having a generally hollow hemispherical shape. The cup 12 is matingly fitted to acetabulum 14. As shown in Figure 12, the prosthesis 10 may include a stem 16 for insertion into opening 20 formed in medullary canal 22. The prosthesis 10 may further include a head 24 which may be secured to proximal end 26 of stem 16. The head 24 may be in mating contact with cup 12 or may as shown in Figure 12 be spaced from the cup 12 by liner 30.

The cup 12 may be made of any suitable, durable material which is compatible with the human anatomy. For strength and durability, typically the cup 12 is made of a metal such as stainless steel, a cobalt chrome alloy or titanium. The cup 12 may alternatively be made of a ceramic.

The liner 30 may be made of any suitable, durable bearing material and is often made of a plastic such as polyethylene, for example, ultrahigh molecular-weight polyethylene. Alternatively, the liner 40 may be made of a metal such as those described relative to the cup or be made of a ceramic.

While the stem 16 may be made of a unitary construction, typically the stem 16 includes a stem portion 32 as well as head 24. The two part construction of the stem 16 provides for easier manufacture and for varying offsets for the prosthesis by utilizing a plurality of heads 24 and/or a plurality of stem portions 32.

The stem portion 32 may be connected to the head 24 in any suitable fashion. For example, the stem portion 32 may include an external tapered portion 34 which mates with an internal tapered portion 36 on the head 24.

As shown in Figure 12, the stem portion 32 may include a proximal stem portion 40, a distal stem portion 42 extending downwardly from the proximal stem portion 40, and a neck portion 44 extending upwardly from the proximal stem portion 40. The proximal stem portion 40 and the distal stem portion 42 are preferably located at least partially within opening 20 formed within the cancellous bone 46 of the medullary canal 22.

The hip prosthesis 10 may be secured to the medullary canal 22 of long bone or femur 50 typically either by a press fit within the medullary canal 22 or with the use of cement mantle 52 which is positioned between the prosthesis 10 and the cancellous bone 46.

In utilizing cement mantle 52, the opening 20 is broached or reamed for example, slightly larger than the stem 16 and a quantity of cement 54 [for example, PMMU (polymethylmethacrylate)] is placed within the opening 20 and the stem 16 is inserted therein. A small uniform layer, for example, 1 to 4 mm of cement, is formed between the stem portion 32 and the femur 50.

When preparing opening 20, femur 50 is resected or cut along resection plane 56 with an appropriate instrument and resected bone 60 is removed. Portions of the prosthesis 10 are thus utilized to replace the resected bone 60. The opening 20 is sized appropriately for a stem 16 and such decisions in the size of the opening depend in part on the size of the patient's femur 50. For example, the opening 20 is sized such that hard or cortical bone 62 is preferably not removed such that only cancellous bone 46 is removed to form the opening 20.

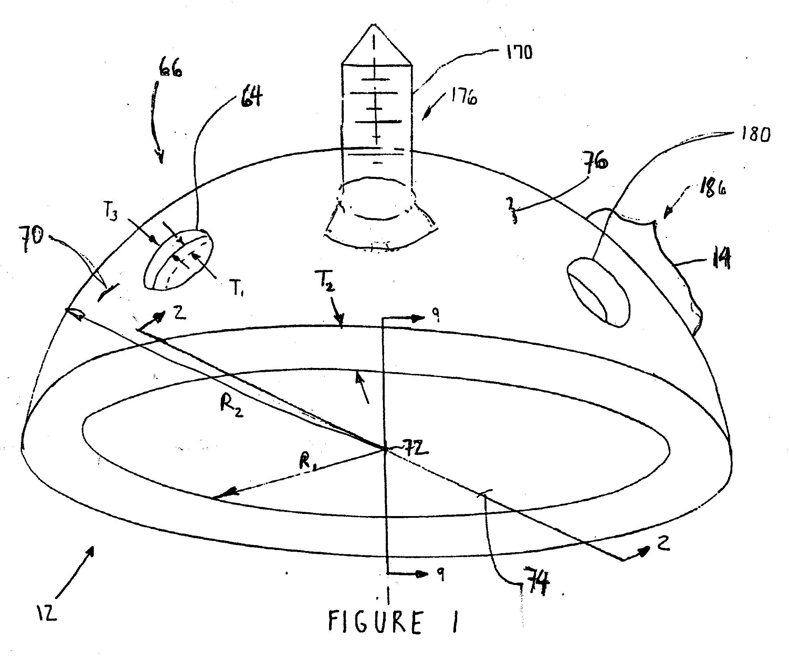

According to the present invention and referring now to Figure 1, the cup 12 is shown in greater detail. As shown in Figure 1, the cup 12 is used for engagement with human anatomy or acetabulum 14. According to the present invention, the cup 12 includes a portion thereof, for example, first portion 64, which has a thickness T₁ at least some location within the first portion 64 which is less than the thickness T₂ of the majority of the cup 12. The reduced thickness of the portion 64 serves to permit at least part of portion 64 to be mechanically separated from the cup 12 to form an opening 66 therein.

The cup 12, as shown in Figure 1, may have a generally hollow hemispherical shape. The cup 12 may be defined by an external surface 70 which is generally hemispherical and defined by radius R₂ extending from origin 72. The cup 12 may further be defined by internal surface 74 of the cup 12. The internal surface 74 may likewise be hemispherical and be defined by radius R₁ extending from origin 72 to internal surface 74 of the cup 12.

The cup 12 may have a thickness T₂ which is approximately equal to R₂ minus R₁. The dimensions R₁ and R₂ may be selected to provide for sufficient strength and rigidity for the cup 12. For example the radius R₂ may be, for example, 17 to 38 mm and the radius R₁ may be, for example, 10 to 16 mm. Correspondingly, the thickness T₂ of the cup may be, for example, from 1 to 28 mm.

The external surface 70 of cup 12 is secured against the human anatomy in the form of acetabulum 14. To assist in the secure placement of the cup 12, the cup 12 may be attached to the acetabulum 14 by bone cement or by a coating which promotes bone ingrowth. Such a suitable coating is porous coating 76.

Various porous coatings have found to be very effective. One particularly effective coating is applied to products which are available from DePuy Orthopaedics Inc under the trade mark Porocoat, and are disclosed in US-3855638.

This porous coating consists of a plurality of small discreet particles of metallic material bonded together at their points of contact with each other to define a plurality of connected interstitial pores in the coating. The particles are of the same metallic material as the metallic material from which the substrate is formed. Examples of suitable material include osteonatic stainless steel, titanium, titanium alloys and cobalt alloys.

The coating 76 may alternatively be a bio-ceramic. Such suitable bio-ceramics include hydroxyapatite or tri-calcium phosphates. Alternatively, the coating 76 may be a combination of bio-ceramics and porous coatings.

The cup 12 may be made of any suitable durable material and may, for example, be made of a stainless steel, a titanium, or a steel alloy, for example cobalt chromium alloy. Alternatively, the cup 12 may be made of ceramic.

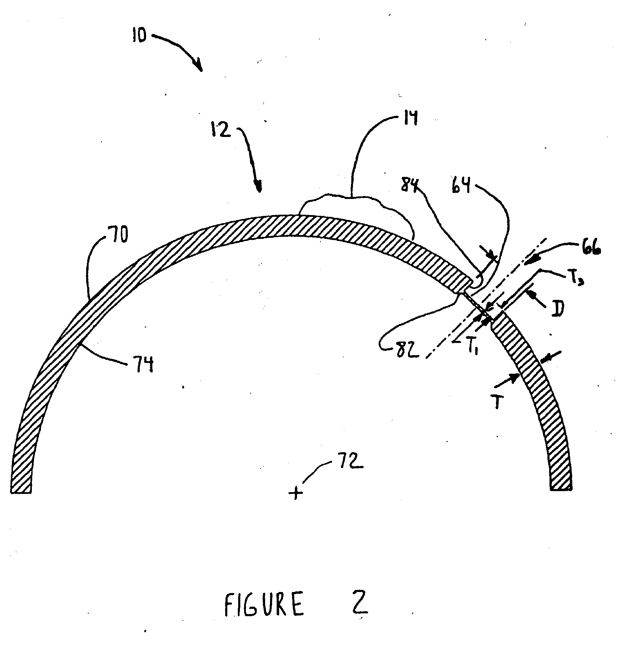

Referring now to Figure 2, the first portion 64 is shown in greater detail. The first portion 64 has a thickness T₁ which is sufficiently thick to prevent synodical fluid from passing there through. For example, the first portion 64 may have a thickness T₁ of, for example, from 0.13 mm to 1.3 mm (0.005 to 0.050 inch). The first portion 64 preferably has a thickness T₁ which is sufficiently thin to permit the first portion 64 to be easily removed to form opening 66.

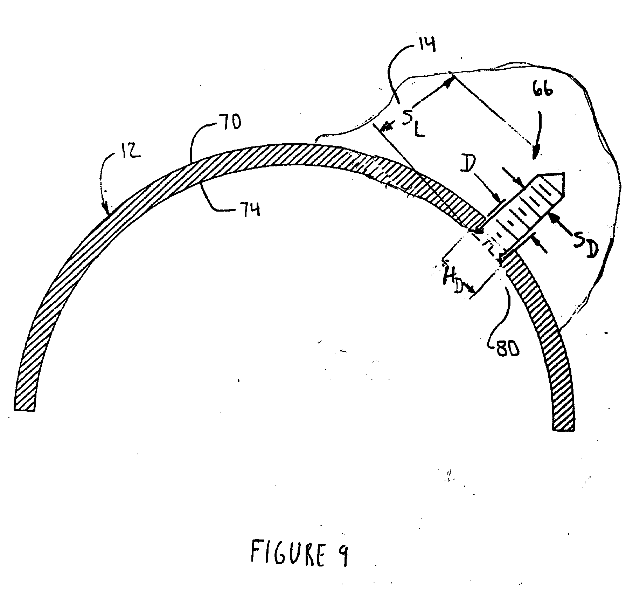

The opening 66 is preferably adapted to fit a suitable standard sized orthopaedic acetabular screw for example screw 80 (see Figure 9). For example as shown in Figure 2 the cup 12 may define chamfer 82 positioned between internal surface 74 and first portion 64. The chamfer 82 may be adapted for cooperation with the screw 80 (see Figure 9). As shown in Figure 2, the cup 12 may include a counterbore 84 formed between external surface 70 and the first portion 64. As shown in Figure 2, the counterbore 84 has a diameter D. Diameter D of counterbore 84 is sized to provide for the insertion of screw 80 (see Figure 9). The counterbore 84 may have a depth T₃ of, for example, 12.7 to 6.4 mm (0.5 to 0.25 inch).

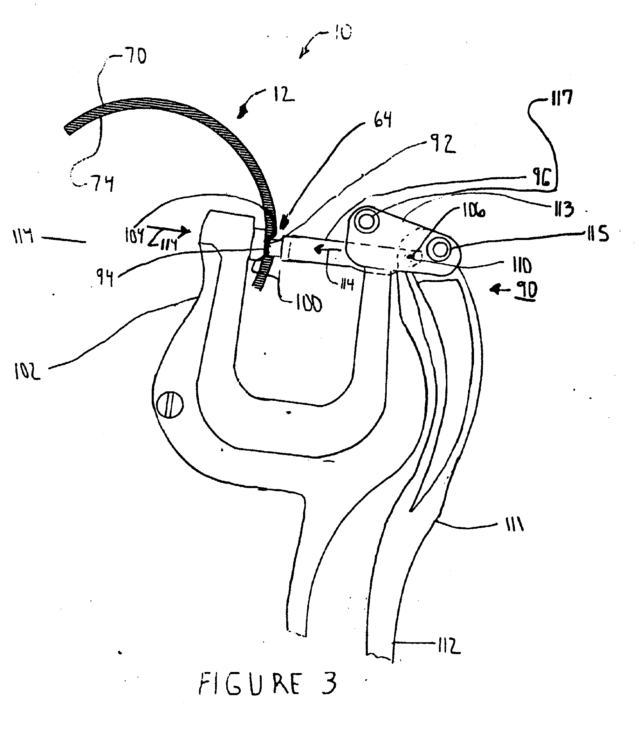

Referring now to Figure 3, a removal tool 90 is shown for removing portion 64 of cup 12. The portion 64 includes a first cup surface 92 which is recessed inwardly from external surface 70 of the cup 12. The portion 64 also defines a second cup surface 94 which is generally parallel to the first cup surface 92 and which second cup surface 94 extends inwardly from internal surface 74 of the cup 12.

As shown in Figure 3, the removal tool 90 includes a first component 96 having a first tool surface 100 which is adapted to conform with the first cup surface 92. Similarly, the tool 90 includes a second component 102 which has a second tool surface 104 adapted to conform with the second cup surface 94. The first component 96 and the second component 102 cooperate with each other to remove the portion 64 of the cup 12 by placing the portion 64 of the cup 12 between the first component 96 and the second component 102 and by advancing the first component 96 toward the second component 102.

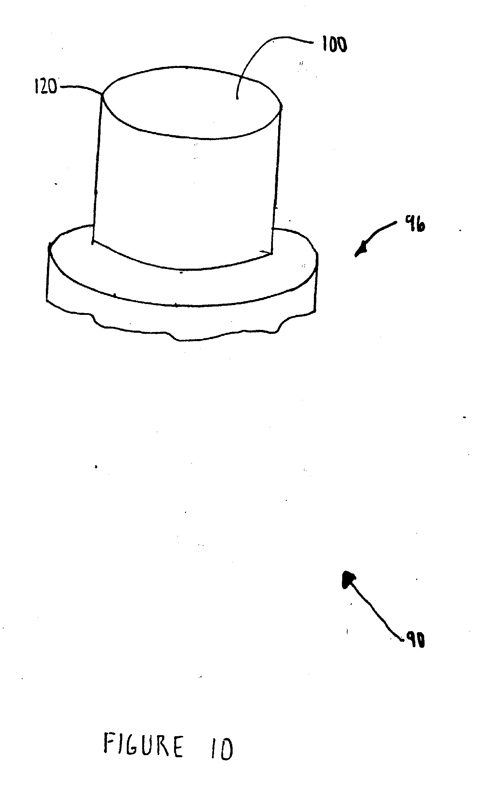

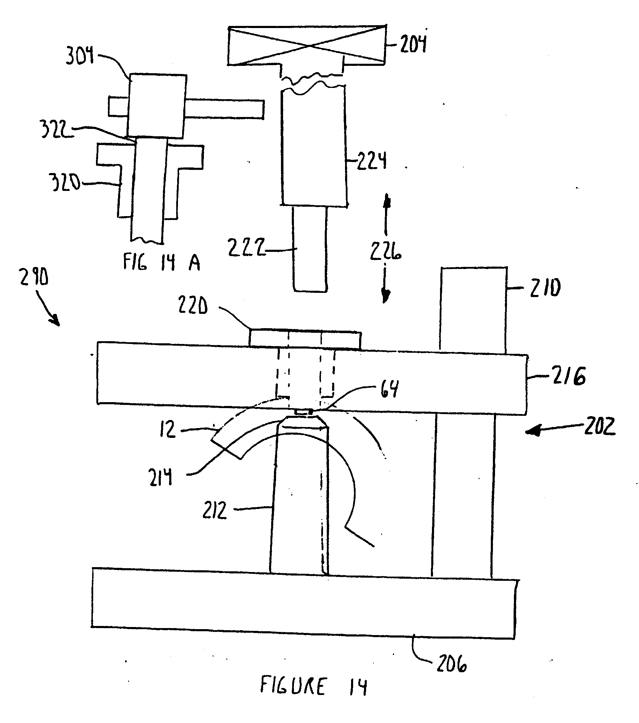

The first component 96 and the second component 102 may have any suitable shape and configuration such that the components 96 and 102 may be advanced toward each other for removal of the portion 64 and correspondingly separated so that the cup 12 may be removed from the tool 90 and so that the tool 90 may be reused. For example, the removal tool 90 may be in the form as shown in Figure 3 of a pliers, an arbor press or a hammer (see Figures 10 and 14).

Referring again to Figure 3, the removal tool 90 may, for example, as shown in Figure 3, include the first component 96 and the second component 102 being slidably moveable with respect to each other. For example, the first component 96 may include an opening 106 to which a first component pin 110 attached to a handle 111 may be matingly fitted. A linkage arm 113 may connect the handle 111 at handle pin 115 to second component 102 at second component pin 117. By squeezing with one's hand on handle end 112 of handle 111 of the removal tool 90 the first tool surface 100 and the second tool surface 104 are moved toward each other in the direction of arrows 114.

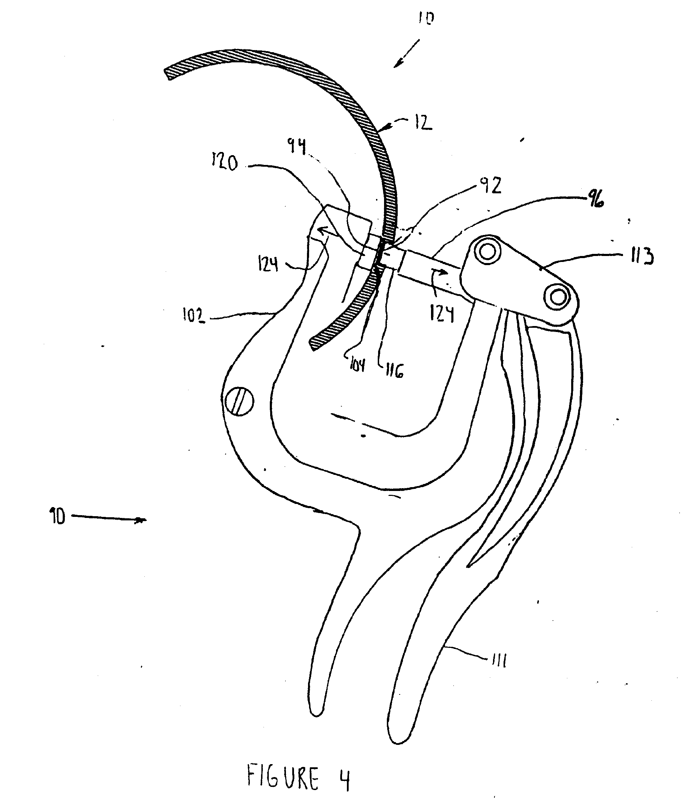

Referring now to Figure 4, the removal tool 90 is shown in greater detail. The first component 96 may as shown in Figure 4 include a first component tip portion 116 which may contact first cup surface 92. Similarly, the second component 102 may include a second component tip portion 120 which includes second tool surface 104 . The first component tip portion 116 and the second component tip portion 120 may be treated to improve the wear characterizations of the surfaces 92 and 94, or the first tip portion 116 and the second tip portion 120 may be made of a material different from that of most of the first component 96 and the second component 102 and may be fastened thereto.

As shown in Figure 4, the tool 90 may include a separating device in the form of spring (not shown) which may be utilized to separate the surfaces 92 and 94 from each other in the direction of arrows 124.

Another embodiment of the present invention is shown as removal tool 290 in Figure 14. Removal tool 290 like removal tool 90 of Figures 3, 4 and 5 is utilized to remove portion 64 from cup 12 (see Figure 2). The removal tool 290 as shown in Figure 14 includes a fixture 202 which cooperates with press 204. The fixture 202 includes, for example, a base 206 to which column 210 and support 212 are attached.

The support 212 is utilized to support cup 12 and the end portion 214 of the support 212 serves as a die or a tool surface. As shown in Figure 14, an arm 216 extends from column 210 and supports guide bushing 220. The guide bushing 220 is utilized to guide punch 222 which is supported by arbor 224 of the press 204. As the press 204 moves arbor 224 upward and downward in the directions of arrows 226 the punch 222 is moved into engagement with the cup 12 to remove the portion 64.

It should be appreciated that the movement of the punch 222 along the guide bushing 220 may be performed by a devise other than press 204. For example, punch 322 supported by bushing 320 may be struck by mallet 304 to remove the portion 64 (see Figure 14A).

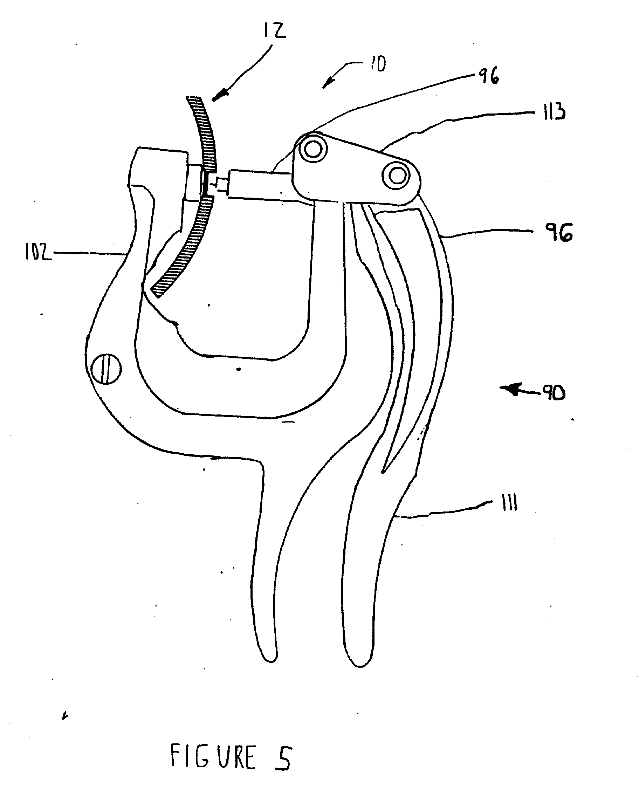

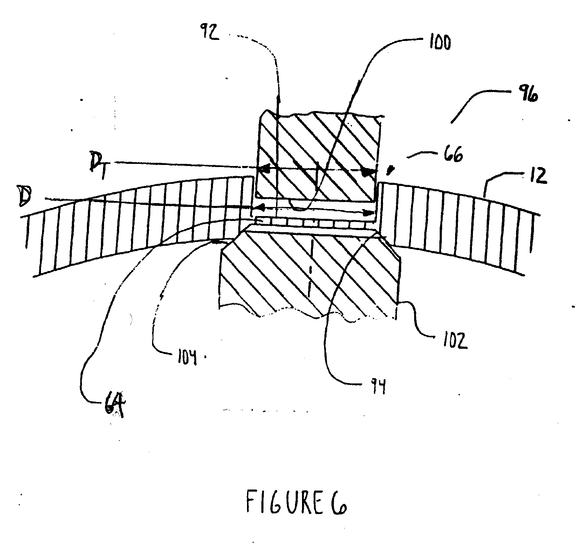

Referring now to Figures 5, 6, 7, 8 and 15, portion 64 of the removal tool 90 and the cup 12 are shown in greater detail. Referring now to Figure 6, the cup 12 is shown positioned on second component 102. The second cup surface 94 presses against second tool surface 104. The first component 96 is positioned above portion 64 and is positioned partially within opening 66 of the cup 12. As shown in Figure 6, the first tool surface 100 may be flat or planar.

As shown in Figure 6, the first component 96 has a cylindrical portion with diameter D_{T} adjacent the first tool surface 100. The diameter D_{T} of the first component 96 is preferably slightly smaller than diameter D of the opening 66. For example, the diameter D_{T} may be about 0 to 0.5 mm (0 to 0.02 inch) smaller than the diameter D of the opening 66. Clearance between the component 96 and the opening 66 permits easy removal of the first component 96 from the opening 66 after the portion 64 has been removed.

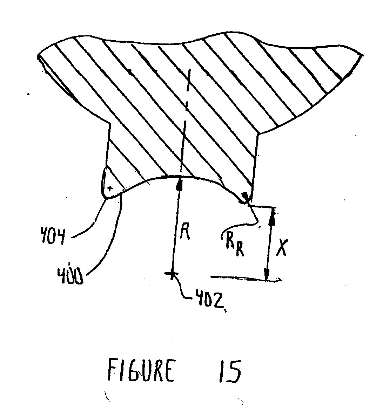

While a flat or planar first tool surface 100 as shown in Figure 6 may be utilized, a non-planer first tool surface 400 as shown in Figure 15 may provide for improved removal of the portion 64. As shown in Figure 15, the first tool surface 400 may be arcuate and defined by radius R extending from origin 402. Origin 402 may be positioned for example, a distance X from outer rim 404 of the first tool surface 400. The outer rim 404 may be somewhat sharp and may have a radius R_{R} of 0 to 0.13 mm (0 to 0.005 inch).

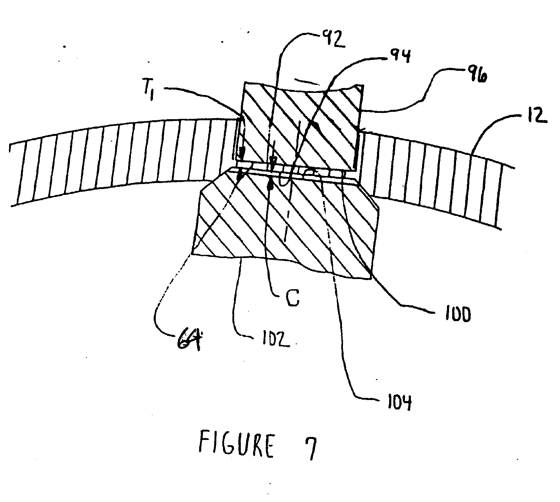

Referring now to Figure 7, the first component 96 is shown in intimate contact with the cup 12. As shown in Figure 7, the first tool surface 100 is in contact with first cup surface 92. As shown in Figure 7, the second tool surface 104 of second component 102 is spaced from the second cup surface 94 to permit a portion of cup 64 to be removed. For example, the second tool surface 104 and the second cup surface 94 may be spaced from each other a distance C which is greater than thickness T₁ of the portion 64.

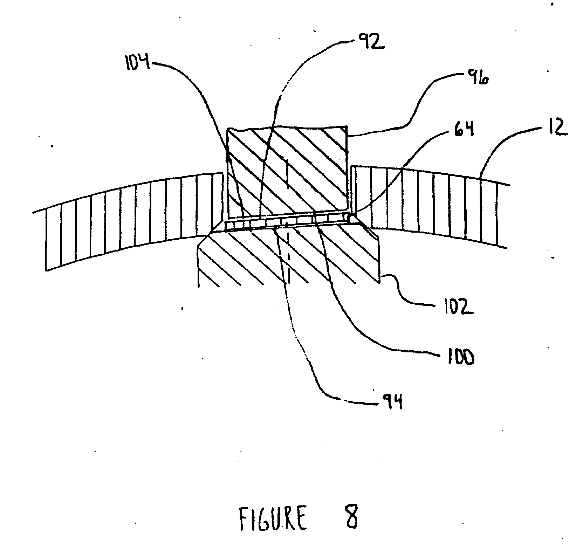

Referring now to Figure 8, first component 96 is shown fully engaged against second component 102. As shown in Figure 8, the first tool surface 100 is in intimate contact with the first cup surface 92 and the second cup surface 94 is in intimate contact with the second tool surface 104. The portion 64 of the cup 12 is now severed from the cup 12. It is in the form of a slug. As can be seen in Figure 8, upon removal of the first component 96 and the second component 102, the slug 64 is no longer part of the cup 12 and may be discarded or disposed of properly.

Referring now to Figure 10, the first component 96 is shown in greater detail. The first component 96 includes the first tool surface 100. The outer rim 120 provides a shearing edge to removes the plug 64.

Referring now to Figure 9, the cup 12 is shown in position in the acetabulum 14. As shown in Figure 9, screw 80 is inserted from the internal surface 74 of the cup 12 through opening 66 in the cup 12. The screw has a screw diameter S_{D} which is slightly less than diameter D of the opening 66.

The screw 80 may be any suitable orthopaedic screw, for example a DePuy Solution System Screw #1250/20/000 may be used. The screw has a head diameter H_{D} and a screw length S_{L} suitable for the application. For example, the screw length S_{L} may be for example from 20 to 70 mm. The screw 80 is fitted and secured to the cup 12 and has a head diameter H_{D} large enough to prevent synodical fluid to enter the internal surface 74 and to prevent debris particles from the prosthesis 10 to enter the acetabulum 14.

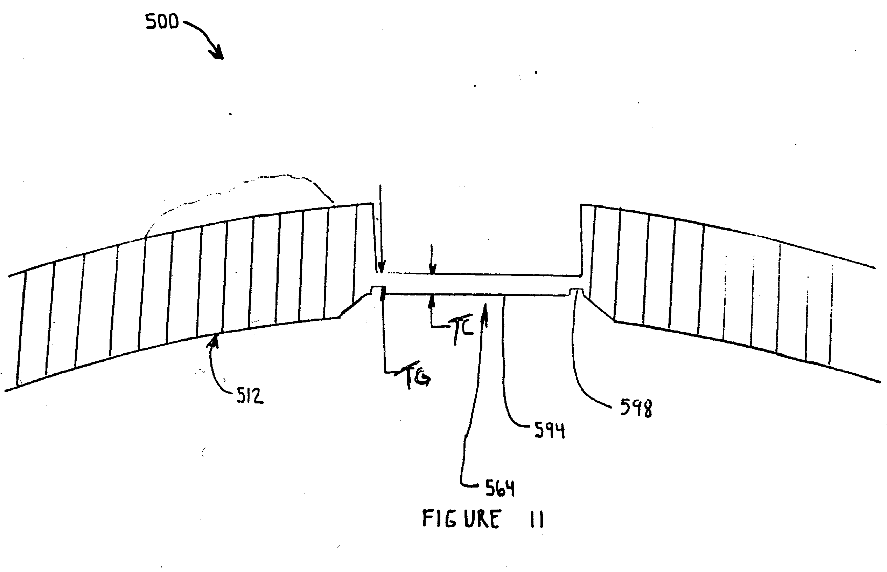

Referring now to Figure 11, an alternate embodiment of the current invention is shown as prosthesis 500. Prosthesis 500 includes cup 512 which is similar to cup 12 of Figures 1-5, but has a portion 564 is slightly different than portion 64 of the cup 12. As shown in Figure 11, the portion 564 of the cup 512 has a second cup surface 594 which is slightly different than second cup surface 94 of the cup 12. For example and as shown in Figure 11, the second cup surface 594 includes a circumferential groove 598 located on the second cup surface 594. The groove 598 results in a thickness of the portion 564 T_{G} which is less than the thickness T_{C} of the central part of portion 564. The utilization of the groove 598 provides for a thicker portion 564 in the central part to prevent the leakage of synodical fluid while the thin grooved area provides for a low removal force when removal of the portion 564 is required.

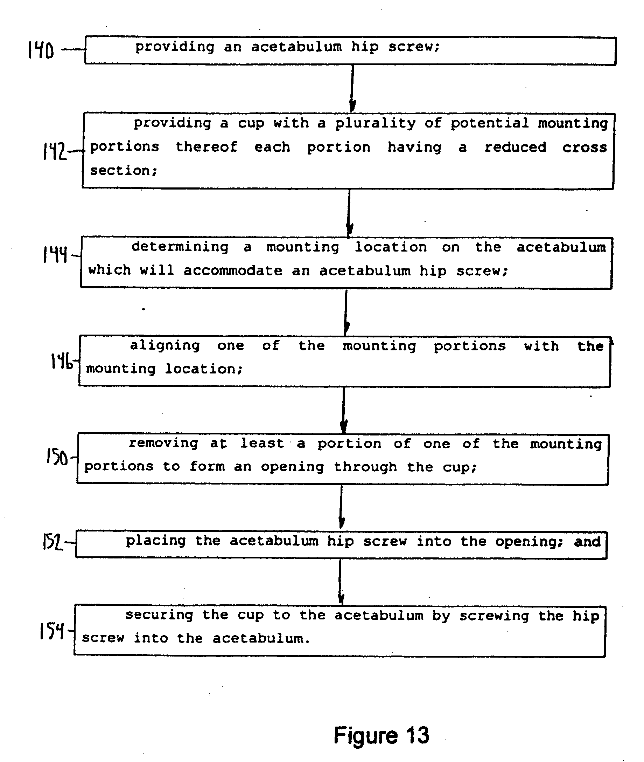

Referring now to Figure 13, a process for performing total hip arthroplasty according to the present invention is shown. The first step 140 includes the step of providing an acetabulum hip screw. Second step 142 includes the step of providing the cup with a plurality of potential mounting portions thereof, each portion having a reduced cross section. Third step 144 provides for determining a mounting location on the acetabulum which will accommodate an acetabulum hip screw. Fourth step 146 provides for aligning one of the mounting portions with the mounting location. Fifth step 150 provides for removing at least a portion of one of the mounting portions to form an opening through the cup. Sixth step 152 provides for placing the acetabulum hip screw into the opening and seventh step 154 provides for securing the cup to the acetabulum by screwing the hip screw into the acetabulum.

Referring again to Figure 1, second portion 170 and third portion 180 are shown. The second portion 170 provides for second opening 176 while the third portion 180 provides for third opening 186. While three openings are shown in Figure 1, it should be appreciated that two, four or any number of portions or openings may be provided with the cup 12. It further should be appreciated that the cup 12 may be provided with both traditional screw holes in the cup and additional areas which utilize the removable portion according to the present invention.

By providing the removable plugs, complete sealing of the screw hole plug in the end of the acetabulum from synodical fluid and polyethylene wear debris is provided.

By utilizing the prosthesis of the present invention with the removable hole plugs, cup prostheses inventory reductions may be possible by having a common plug that may be used for a variety of situations in which different hole locations are required.

## Claims

1. A cup for engagement with the human anatomy for use in an articulating hemiarthroplasty prosthesis for implantation into the human anatomy, said cup comprising a portion thereof having a reduced thickness to permit at least a portion thereof to be mechanically separated from the cup to form an opening therethrough.

2. The cup of claim 1, wherein the portion of said cup having a reduced thickness is adapted to block the flow of synodical fluid therethrough.

3. The cup of claim 1, wherein the portion of said cup having a reduced thickness has a generally cylindrical shape.

4. The cup of claim 1, wherein the portion of said cup having a reduced thickness has a thickness of around 2.5 mm (0.10 inch) or less.

5. The cup of claim 1, wherein the portion of said cup having a reduced thickness is adapted to be sheared by a punch tool.

6. The cup of claim 1, further comprising:
a stem;
a head operably associated with said stem; and
a liner positioned between said cup and said head.

7. The cup of claim 1, further comprising a second portion thereof spaced from the first mentioned portion thereof, said second portion having a reduced thickness to permit at least a portion thereof to be mechanically separated from the cup to form an opening therethrough whereby at least one of the first mentioned portion and the second portion may be selected to be mechanically separated from the cup to provide an opening for securing the cup to the human anatomy.

8. A tool for use with a articulating hemiarthroplasty cup prosthesis to remove a portion of the cup, the portion having a first cup surface and a second opposed cup surface, said tool comprising:
a first component having a first tool surface adapted to conform with the first cup surface; and
a second component having a second tool surface adapted to conform with the second cup surface, said first component and said second component adapted to cooperate with each other to remove the portion of the cup by placing the portion of the cup between said first component and said second component and by advancing said first component toward said second component.

9. The tool of claim 8, wherein said first component and said second component are pivotally attached to each other.

10. A hip prosthesis for implantation into the human anatomy, comprising a cup for engagement with the acetabulum, said cup including a portion thereof having a reduced thickness to permit at least a portion thereof to be mechanically separated from the cup to form an opening therethrough, the portion of said cup having a reduced thickness being adapted to block the flow of synodical fluid therethrough.

11. The hip prosthesis of claim 10, wherein the portion of said cup having a reduced thickness has a generally cylindrical shape.

12. The hip prosthesis of claim 10, further comprising:
a stem;
a head operably associated with said stem; and
a liner positioned between said cup and said head.

13. The prosthesis of claim 10, wherein the portion of said cup having a reduced thickness is adapted to be sheared by a punch tool.

14. The prosthesis of claim 10, further comprising a second portion thereof spaced from the first mentioned portion thereof, said second portion having a reduced thickness to permit at least a portion thereof to be mechanically separated from the cup to form an opening therethrough whereby at least one of the first mentioned portion and the second portion may be selected to be mechanically separated to provide an opening for securing the cup to the acetabulum.
